(19) Europäisches Patentamt
European Patent Office
Office européen des brevets

(11) Numéro de publication : **0 092 513**
**B1**

(12) **FASCICULE DE BREVET EUROPÉEN**

(45) Date de publication du fascicule du brevet : **12.11.86**

(51) Int. Cl.⁴ : **A 61 F 9/00**, G 02 C 7/04

(21) Numéro de dépôt : **83810138.4**

(22) Date de dépôt : **07.04.83**

# (54) Verre de contact.

(30) Priorité : **16.04.82 CH 2317/82**
**24.05.82 FR 8209097**

(43) Date de publication de la demande :
**26.10.83 Bulletin 83/43**

(45) Mention de la délivrance du brevet :
**12.11.86 Bulletin 86/46**

(84) Etats contractants désignés :
**DE GB IT NL**

(56) Documents cités :
**EP-A- 0 030 210**
**FR-A- 2 248 814**
**FR-A- 2 255 616**
**US-A- 3 467 099**
**US-A- 3 726 587**

(73) Titulaire : **LASAG AG**
**Bernstrasse 11**
**CH-3600 Thun (CH)**

(72) Inventeur : **Riquin, Didier**
**9, Rue Bridaine**
**F-75017 Paris (FR)**

(74) Mandataire : **Gresset, Jean et al**
**SMH Société Suisse de Microélectronique et d'Horlogerie S.A. Département Brevets et Licences 6, Faubourg du Lac**
**CH-2501 Bienne (CH)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**EP 0 092 513 B1**

Jouve, 18, rue St-Denis, 75001 Paris, France

**Description**

La présente invention concerne un verre de contact pour l'observation et le traitement par un faisceau de rayonnement cohérent d'un point de travail situé dans un œil sur l'axe optique du verre, ce verre de contact comprenant une surface d'entrée sphérique ayant un point de Weierstrass réel et un point de Weierstrass virtuel situés tous deux sur ledit axe optique, une surface de contact destinée à être appliquée sur la cornée de l'œil, et un corps situé entre la surface d'entrée et la surface de contact, les centres optiques desdites surfaces étant situés tous deux sur ledit axe optique.

Différentes affections de l'œil comme par exemple certains glaucomes, la cataracte ou encore les troubles résultant de la présence de membranes peuvent nécessiter pour leur opération ou la préparation de cette opération la mise en œuvre d'un faisceau laser de très forte densité d'énergie dans certaines régions de l'œil. A cet effet, on utilise des installations de traitement ophtalmologique du type de celle décrite dans la demande de brevet EP-A-0 030 210 au nom de la demanderesse. Ces installations permettent de diriger vers l'œil un faisceau laser constitué le plus souvent d'impulsions très brèves mais de très forte intensité délivrées par exemple par un laser dont la partie active comporte un grenat d'yttrium et d'aluminium dopé au néodyme, communément appelé laser Nd-YAG, fonctionnant en mode Q-Switch. L'ouverture du faisceau à la sortie de l'installation est de l'ordre de 16°.

Bien entendu, on peut envisager de diriger ce faisceau directement sur l'œil sans utiliser de verre de contact, et cela notamment pour atteindre l'iris ou le voisinage du cristallin par un tir effectué selon une direction parallèle à l'axe de l'œil. L'ouverture moindre du faisceau garantit alors des aberrations d'ouverture faibles à l'entrée dans l'œil, mais elle engendre également de nombreux désavantages. En particulier, les risques d'atteindre des zones autres que celle réellement visée sont augmentés, la localisation du phénomène de claquage optique mis à profit pour traiter cette zone étant moins fine. La tache de focalisation du faisceau sur la cible est également plus grande.

Les verres de contact pouvant être utilisés sont divers. Nous citerons pour mémoire les trois catégories suivantes :

Les verres du type Goldmann sont réalisés d'une pièce, le plus souvent en un matériau organique, par exemple acrylique, et ils présentent une surface d'entrée plane ou très légèrement sphérique. A l'origine, ils étaient prévus essentiellement pour l'examen du fond de l'œil, mais ils sont aussi utilisés pour des interventions dans la chambre antérieure de l'œil. Ces verres de contact ne résolvent pas les problèmes liés à l'ouverture du faisceau qu'ils diminuent encore par rapport à la solution précédente, et de plus ils introduisent de fortes aberrations au niveau de leur face d'entrée. Leur avantage essentiel est d'assurer une très bonne sécurité et le confort du patient au niveau de l'interface verre de contact-œil en raison du très bon état de surface que l'on peut obtenir avec les matériaux utilisés, cet état de surface évitant la formation de micro-claquages à cet interface. Par contre, l'adjonction sur leur surface d'entrée d'un revêtement anti-reflet à la fois pour les longueurs d'onde du faisceau de traitement (1,06 $\mu$ pour le laser Nd-YAG) et dans le visible présente des difficultés non encore parfaitement maîtrisées.

Les verres du type Abraham sont constitués par une lentille d'entrée en verre minéral accolée de manière décentrée à une lentille de contact du type Goldmann. Ces verres, utilisables pour l'iridectomie seulement, permettent d'ouvrir le faisceau de traitement ce qui en garantit une meilleure focalisation. De plus, la face d'entrée peut être aisément traitée. Toutefois, aux aberrations au niveau de la surface d'entrée s'ajoute une aberration de coma au niveau du dioptre d'entrée sur l'œil, due à l'effet conjugué d'une plus grande ouverture, et d'un décentrement de la lentille.

Les verres du type Roussel développés plus récemment et décrits par exemple dans la demande de brevet EP-A-0 059 159 au nom de la demanderesse sont réalisés d'une pièce, en verre minéral, et ils comportent une face latérale formant miroir. La surface d'entrée, sphérique, forme une surface d'onde pour le faisceau incident dont l'ouverture n'est pas modifiée. L'avantage essentiel de ces verres est de permettre de réduire considérablement les aberrations, en raison de leur meilleure géométrie. Ils sont en outre peu sensibles aux rotations du verre. Par contre, leur utilisation est relativement délicate en raison de la présence du miroir. L'inclinaison du faisceau de traitement par rapport à l'axe de l'œil diminue par ailleurs le rendement du tir. Enfin, en raison des problèmes d'interface déjà explicités plus haut, ils ne garantissent pas une sécurité optimale à la jonction verre minéral-cornée.

La demande de brevet FR-A-2 248 814 décrit un verre de contact comprenant une lentille ayant un indice de réfraction très élevé, supérieur à 1,65, dont la face d'entrée est sphérique et a un faible rayon de courbure, et dont l'épaisseur est supérieure à ce rayon de courbure. Ce verre de contact présente l'inconvénient d'introduire une aberration relativement importante, ce qui diminue également l'efficacité du faisceau laser de traitement.

Le but de la présente invention est de proposer un nouveau verre de contact qui réalise un excellent compromis entre les exigences parfois contradictoires auxquelles ces dispositifs sont soumis. La structure de ce verre de contact est déterminée de manière à ouvrir le faisceau de traitement incident, à limiter les aberrations au minimum, à permettre d'effectuer des tirs aussi perpendiculairement au tissu visé que possible, à assurer la sécurité et le confort du patient à l'interface verre-cornée, et ceux du médecin à l'entrée du faisceau dans le verre (traitement anti-réfléchissant), et cela sans imposer au médecin un positionnement trop critique du verre sur la cornée.

Ce but est atteint par le verre de contact revendiqué.

L'avantage résultant de l'utilisation des points de Weierstrass est bien connu en optique puisqu'il s'agit de points conjugués aplanétiquement, travaillant pour un grandissement égal à l'indice du matériau utilisé pour la lentille d'entrée (de l'ordre de 1,5). Les aberrations d'ouverture à l'entrée du verre de contact sont supprimées du fait de ce mode de fonctionnement, et la focalisation du faisceau est importante. Dans le cas de la mise en œuvre d'au moins deux lentilles accolées, il est par ailleurs possible d'utiliser pour la lentille d'entrée un verre minéral, et pour la lentille de contact un verre organique.

L'invention sera bien comprise à la lecture de la description suivante faite en liaison avec les dessins joints, parmi lesquels :

les figures 1 et 2 sont des représentations schématiques permettant d'illustrer de façon générale les conditions de fonctionnement et d'utilisation d'un verre de contact selon l'invention ; et

les figures 3 et 4 sont des vues en coupe de deux verres de contact et de l'œil auquel ils sont appliqués, selon deux modes de réalisation particuliers de l'invention.

Dans la figure 1, on a représenté en coupe et de façon schématique le verre de contact généralement référencé 1 appliqué sur la cornée 2 d'un œil 3. Le verre de contact 1 comprend une lentille d'entrée 4 accolée à une lentille de contact 5 dont la surface de contact est destinée à être appliquée sur la cornée 2. Les axes optiques des lentilles 4 et 5 sont confondus et désignés par la référence 6. Les intersections de l'axe unique 6 avec les dioptres air-lentille d'entrée, lentille d'entrée-lentille de contact et lentille de contact-œil sont représentées respectivement par les points Se, Sc et So. Les épaisseurs au centre des lentilles d'entrée 4 et de contact 5 sont représentées respectivement par les grandeurs ee et ec. Sur l'axe 6 on a également représenté le point de travail T dont la distance au point So est définie par la grandeur d. Dans les explications qui suivent, les symboles Re, Rc et Ro représentent respectivement les rayons de courbure des dioptres air-lentille d'entrée, lentille d'entrée-lentille de contact et lentille de contact-œil. De même les indices de réfraction de la lentille d'entrée, de la lentille de contact et de l'œil sont désignés respectivement par les symboles Ne, Nc et No.

Selon l'invention, la surface d'entrée sphérique 8 de la lentille d'entrée 4 travaille pour les points de Weierstrass A et A', tandis que le reste du verre de contact et les parties de l'œil traversées par le faisceau amènent l'image du point A' au point de travail T. En particulier, le point A" représente l'image du point A' par le dioptre lentille d'entrée-lentille de contact.

Compte tenu de cette structure, on peut alors écrire les relations suivantes :

pour le dioptre lentille de contact-œil

$$\frac{Nc}{x} = \frac{No}{d} + \frac{Nc - No}{Ro} \quad \text{avec } x = SoA''$$

pour le dioptre lentille d'entrée-lentille de contact

$$\frac{Ne}{ScA'} = \frac{Nc}{x + ec} + \frac{Ne - Nc}{Rc}$$

pour le dioptre air-lentille d'entrée

$$SeA' = ScA' + e_e$$

$$SeA' = \frac{(Ne + 1)}{Ne} Re \qquad \text{Relations de Weierstrass}$$

$$SeA = (Ne + 1)\ Re$$

La combinaison de ces relations permet alors d'obtenir la relation générale qui lie les caractéristiques géométriques et optiques des différentes lentilles et de l'œil pour un verre de contact conforme à la figure 1.

$$Re = \frac{Ne^2}{Ne + 1}\left(\frac{1}{\dfrac{Nc}{\dfrac{Nc}{\dfrac{No}{d} + \dfrac{Nc - No}{Ro}} + ec} + \dfrac{Ne - Nc}{Rc}} + \frac{ee}{Ne}\right)$$

Les différentes grandeurs intervenant dans la relation générale peuvent varier dans les limites suivantes :

7 mm $\leq$ Ro $\leq$ 9 mm Tabulae Biologicae
1,33 $\leq$ No $\leq$ 1,34 Tabulae Biologicae
0,5 mm $\leq$ ec $\leq$ 30 mm Tabulae Biologicae

$1,3 \leq Nc \leq 2,0$ Tabulae Biologicae
$2 \text{ mm} \leq Re \leq 100 \text{ mm}$ Tabulae Biologicae
$1,3 \leq Ne \leq 2,0$ Tabulae Biologicae
$0,5 \text{ mm} \leq ee \leq 30 \text{ mm}$ Tabulae Biologicae
$0,5 \text{ mm} \leq d \leq 30 \text{ mm}$ Tabulae Biologicae

La valeur de Rc est quelconque.

Bien entendu, les relations précédentes sont valables pour des verres de contact constitués par l'accolement de deux lentilles seulement. Des relations analogues pourraient être établies pour le cas où l'une ou l'autre de ces lentilles, ou même les deux, seraient elles-mêmes formées par l'accolement de lentilles élémentaires. Une telle construction ne sortirait pas du cadre de la présente invention. De la même façon il est clair que le verre de contact pourrait n'être constitué que par une lentille unique, d'une pièce. Dans ce cas, la surface d'accouplement des deux lentilles de la construction décrite plus haut pourrait être considérée comme une surface fictive séparant le verre de contact en deux portions situées de part et d'autre de cette surface fictive, et présentant le même indice. La relation précédente se transforme alors simplement en égalant les indices Nc et Ne, et en annulant la grandeur ec, ee représentant l'épaisseur totale du verre.

Le schéma de la figure 1 se rapportait au cas où le point de travail se trouve placé dans l'axe même de l'œil. Comme on le verra plus loin, un certain nombre d'affections peuvent nécessiter d'intervenir dans d'autres régions de l'œil. Dans cette hypothèse, le verre de contact devra être maintenu sur la cornée de manière que son axe optique 6 forme avec l'axe de l'œil 10 un angle $\alpha$ (figure 2). En désignant par h la distance du point de travail T de l'axe 10, et par D la profondeur de la projection T' du point T sur l'axe 10. Dans ces conditions, les relations suivantes peuvent être établies :

$$h = -(Ro - D) \operatorname{tg} \alpha$$

$$d = Ro - \frac{Ro - D}{\operatorname{Cos} \alpha}$$

Pour une opération telle que l'iridectomie, la valeur de D est comprise entre 3,4 mm et 4,2 mm (Tabulae Biologicae). L'inclinaison peut être choisie entre 0 et 60° selon la position du point T.

A titre d'exemples non limitatifs de modes de réalisations particuliers de l'invention, on décrira dans ce qui suit deux verres de contact réalisés à partir de lentilles de contact disponibles sur le marché. A ces lentilles ont été accolées des lentilles d'entrée dimensionnées de manière que les verres de contact ainsi formés soient conformes à l'invention.

Ainsi, le verre de contact 1 de la figure 3, est particulièrement adapté pour l'iridectomie en vue par exemple du traitement des glaucomes à angle fermé ou à bloc pupillaire, pour la perforation de la capsule antérieure du cristallin ou encore l'ablation de membranes dans la chambre antérieure de l'œil. Dans l'utilisation représentée dans la figure 3, le point de travail T se trouve sur l'iris 11 de l'œil 3. La lentille de contact 5 est une lentille vendue par la maison Haag-Streit AG à Berne (Suisse) sous le numéro 902, à laquelle on a enlevé le miroir qu'elle comporte normalement. Les caractéristiques de cette lentille sont les suivantes :

$$Nc = 1,492, \quad ec = 13,2 \text{ mm} ; \quad Rc = \infty.$$

La surface de sortie ou de contact de cette lentille, qui doit être appliquée contre la cornée, est sphérique concave et son rayon de courbure est de 7,4 mm.

Sur la face plane de cette lentille est accolée une lentille d'entrée 4, plan-convexe et en verre BK7, d'un diamètre de 19 mm environ et dont les caractéristiques sont les suivantes :

$$Ne = 1,507 ; \quad ee = 6,10 \text{ mm} ; \quad Re = 13,03 \text{ mm} ; \quad Rc = \infty.$$

Comme on le voit sur la figure, l'ouverture d'un faisceau de traitement est amenée par le dioptre air-lentille d'entrée d'une valeur de 16° à la valeur de 24°.

La surface d'entrée 8 a été traitée anti-reflet pour les longueurs d'onde du faisceau de traitement (1,06 $\mu$) et pour le rayonnement visible.

L'inclinaison du verre par rapport à l'axe 10 de l'œil est de l'ordre de 40° à 50°. Les paramètres de la lentille d'entrée ont été calculés pour les valeurs 48° et D = 3,6 mm.

Les aberrations introduites par un tel verre se limitent à celles créées par le dioptre plan entre les deux lentilles accolées présentant une différence d'indice de 1 % environ seulement, et les dioptres sphériques lentille de contact-cornée et cornée-humeur acqueuse de l'œil. Dans de bonnes conditions de travail, ces aberrations restent très faibles, notamment lorsque l'axe du faisceau optique passe par le centre de courbure de ces dioptres. A ce sujet on notera également que l'utilisateur du verre de contact aura intérêt à respecter autant que possible les conditions d'orientation théoriques du verre, mais qu'il garde néanmoins une certaine liberté de manœuvre.

Le verre de contact de la figure 4 est, quant à lui, plus particulièrement destiné aux interventions dans

la région post-cristallienne, par exemple pour l'ablation de membranes, la perforation de la capsule postérieure du cristallin ou la destruction du cristallin dans le traitement de la cataracte.

La lentille de contact 5 est une lentille vendue par la maison Haag-Streit déjà mentionnée sous le numéro 901, qui comporte une surface de sortie ou de contact destinée à être appliquée sur la cornée sphérique concave, de rayon de courbure 7,4 mm. Les autres paramètres de cette lentille sont

$$Ne = 1{,}492\ ;\quad ee = 1{,}17\ mm\ ;\quad Rc = 150\ mm.$$

A cette lentille de contact 5 est accolée une lentille d'entrée 4 du type mécanique convergent en verre BK7 présentant les caractéristiques suivantes :

$$Ne = 1{,}507\ ;\quad ee = 3{,}65\ mm\ ;\quad Re = 8{,}01\ mm\ ;\quad Rc = 150\ mm.$$

Comme on le voit sur la figure 4, la configuration du verre de contact est telle que le point de travail T se trouve situé entre les points de Weierstrass A et A', à une distance d de 8,5 mm de la surface de la cornée. Les aberrations créées par ce verre sont extrêmement faibles.

D'une façon générale, les verres de contact selon l'invention se caractérisent par une très grande amélioration de leur sécurité d'utilisation par rapport aux dispositifs antérieurs. Ainsi, l'augmentation de l'ouverture du faisceau de traitement conduit à une diminution correspondante de la tache de focalisation, à une augmentation de la densité d'énergie en ce point permettant de diminuer l'énergie du faisceau nécessaire pour le claquage optique, à une meilleure localisation du claquage optique dont la stabilité sera meilleure, c'est-à-dire que pour différentes impulsions successives il se produira toujours au même endroit ce qui augmente l'efficacité du traitement. De plus, le faisceau étant plus ouvert, l'énergie susceptible de tomber sur la rétine ou les régions de l'œil situées avant ou après le point de travail sera également moindre.

**Revendications**

1. Verre de contact (1) pour l'observation et le traitement par un faisceau de rayonnement cohérent d'un point de travail (T) situé dans un œil (3) sur l'axe optique (6) du verre (1), ce verre de contact (1) comprenant une surface d'entrée sphérique (8) ayant un point de Weierstrass réel (A') et un point de Weierstrass virtuel (A) situés tous deux sur ledit axe optique (6), une surface de contact destinée à être appliquée sur la cornée (2) de l'œil (3), et un corps situé entre la surface d'entrée (8) et la surface de contact, les centres optiques desdites surfaces étant situés tous deux sur ledit axe optique (6), caractérisé en ce que le rayon de courbure (Re) de la surface d'entrée (8) a une valeur telle que le corps du verre, la surface de contact et les parties de l'œil (3) traversées par ledit faisceau forment, ensemble, une image du point de Weierstrass réel (A') située à l'emplacement du point de travail (T).

2. Verre de contact selon la revendication 1, caractérisé en ce qu'il est constitué par au moins une lentille d'entrée (4) accolée à une lentille de contact (5), les axes optiques desdites lentilles (4, 5) étant confondus.

3. Verre de contact selon la revendication 2, caractérisé en ce que le rayon de courbure (Re) de la surface d'entrée (8) est défini par la relation suivante :

$$Re = \frac{Ne^2}{Ne + 1}\left(\frac{1}{\dfrac{Nc}{\dfrac{Nc}{\dfrac{No}{d} + \dfrac{Nc - No}{Ro}} + ec} + \dfrac{Ne - Nc}{Rc}} + \frac{ee}{Ne}\right)$$

dans laquelle

Ne, Nc et No représentent respectivement les indices de réfraction de la lentille d'entrée (4), de la lentille de contact (5) et de l'œil (3) ;

Re, Rc et Ro représentent respectivement les rayons de courbure de la surface d'entrée (8), d'un dioptre formé par la lentille d'entrée (4) et la lentille de contact (5) et de la surface de contact ;

d représente la distance entre le point de travail (T) et la surface de contact, dans l'axe optique (6) du verre (1) ; et

ee et ec représentent les distances dans l'axe optique (6) du verre (1), entre la surface d'entrée (8) et le dioptre et, respectivement, le dioptre et la surface de contact.

4. Verre de contact selon la revendication 3, caractérisé en ce que le rayon de courbure Re est défini par ladite relation, dans laquelle

$7\ mm \leqq Ro \leqq 9\ mm$ Tabulae Biologicae
$1{,}33 \leqq No \leqq 1{,}34$ Tabulae Biologicae
$0{,}5\ mm \leqq ec \leqq 30\ mm$ Tabulae Biologicae

1,3 ≦ Nc ≦ 2,0 Tabulae Biologicae
2 mm ≦ Re ≦ 100 mm Tabulae Biologicae
1,3 ≦ Ne ≦ 2,0 Tabulae Biologicae
0,5 mm ≤ ee ≤ 30 mm Tabulae Biologicae
0,5 mm ≤ d ≤ 30 mm Tabulae Biologicae

5. Verre de contact selon l'une quelconque des revendications 2 à 4 précédentes, caractérisé en ce que la lentille d'entrée (4) est réalisée en verre minéral, et en ce que la lentille de contact (5) est réalisée en un matériau organique.

6. Verre de contact selon la revendication 5, caractérisé en ce que la surface d'entrée (8) est munie d'un revêtement anti-reflet.

**Claims**

1. Contact lens (1) for observation and treatment by a beam of coherent radiation of a working point (T) situated in an eye (3) on the optical axis (6) of the lens (1), such contact lens (1) comprising a spherical entry surface (8) having a real Weierstrass point (A') and a virtual Weierstrass point (A) both situated on the optical axis (6), a contact surface intended to be applied to the cornea (2) of the eye (3) and a body situated between the entry surface (8) and the contact surface, the optical centers of said surfaces both being situated on said optical axis (6) characterized in that the radius of curvature (Re) of the entry surface (8) has a value such that the body of the lens, the contact surface and the parts of the eye (3) traversed by said beam together form an image of the real Weierstrass point (A') situated at the location of the working point (T).

2. Contact lens according to claim 1 characterized in that it is constituted by at least one entry lens (4) attached to a contacting lens (5), the optical axes of said lenses (4, 5) being merged into one another.

3. Contact lens according to claim 2 characterized in that the radius of curvature of the entry surface (8) is defined by the following relationship :

$$Re = \frac{Ne^2}{Ne + 1}\left(\frac{1}{\dfrac{Nc}{\dfrac{Nc}{\dfrac{No}{d} + \dfrac{Nc - No}{Ro}} + ec} + \dfrac{Ne - Nc}{Rc}} + \frac{ee}{Ne}\right)$$

in which

Ne, Nc and No represent respectively the indices of refraction of the entry lens (4), the contacting lens (5) and the eye (3) ;

Re, Rc and Ro represent respectively the radii of curvature of the entry surface (8), of a diopter formed by the entry lens (4) and the contacting lens (5) and of the contact surface ;

d represents the distance between the working point (T) and the contact surface along the optical axis (6) of the lens (1) ; and

ee and ec represent the distances along the optical axis (6) of the lens (1) between the entry surface (8) and the diopter and, respectively the diopter and the contact surface.

4. Contact lens according to claim 3 characterized in that the radius of curvature Re is defined by said relationship in which :

7 mm. ≦ Ro ≦ 9 mm Tabulae Biologicae
1,33 ≦ No ≦ 1,34 Tabulae Biologicae
0,5 mm ≦ ec ≦ 30 mm Tabulae Biologicae
1,3 ≦ Nc ≦ 2,0 Tabulae Biologicae
2 mm ≦ Re ≦ 100 mm Tabulae Biologicae
1,3 ≦ Ne ≦ 2,0 Tabulae Biologicae
0,5 mm ≤ ee ≤ 30 mm Tabulae Biologicae
0,5 mm ≤ d ≤ 30 mm Tabulae Biologicae

5. Contact lens according to any of preceding claims 2 to 4 characterized in that the entry lens (4) is formed of mineral glass and that the contacting lens (5) is formed from an organic material.

6. Contact lens according to claim 5 characterized in that the entry surface (8) is provided with an antireflection coating.

**Patentansprüche**

1. Kontaktglas (1) für die Beobachtung und die Behandlung mittels eines kohärenten

Strahlenbündels eines Arbeitspunktes (T), der sich in einem Auge (3) auf der optischen Achse (6) des Glases (1) befindet, welches Kontaktglas (1) eine sphärische Eintrittsfläche (8) mit einem reellen Weierstrass-Punkt (A') und einem virtuellen Weierstrass-Punkt (A) aufweist, die sich beide auf der genannten optischen Achse (6) befinden, eine Kontaktfläche, dazu bestimmt, auf die Cornea (2) des Auges (3) gesetzt zu werden und einen Korpus, der sich zwischen der Eintrittsfläche (8) und der Kontaktfläche befindet, wobei die optischen Zentren der genannten Oberflächen sich beide auf der genannten optischen Achse (6) befinden, dadurch gekennzeichnet, daß der Krümmungsradius (Re) der Eintrittsfläche (8) einen Wert derart besitzt, daß der Korpus des Glases die Kontaktfläche und die Teile des Auges (3), die von dem genannten Strahlenbündel durchsetzt werden, gemeinsam eine Abbildung des reellen Weierstrass-Punktes (A') bilden, die sich an der Stelle des Arbeitspunktes (T) befindet.

2. Kontaktglas nach Anspruch 1, dadurch gekennzeichnet, daß es aus mindestens einer Eintrittslinse (4) besteht, die auf eine Kontaktlinse (5) aufgeklebt ist, wobei die optischen Achsen der genannten Linsen (4, 5) zusammenfallen.

3. Kontaktglas nach Anspruch 2, dadurch gekennzeichnet, daß der Krümmungsradius (Re) der Eintrittsfläche (8) durch die folgende Beziehung definiert ist :

$$Re = \frac{Ne^2}{Ne + 1}\left(\frac{1}{\dfrac{Nc}{\dfrac{Nc}{\dfrac{No}{d} + \dfrac{Nc - No}{Ro}} + ec} + \dfrac{Ne - Nc}{Rc}} + \frac{ee}{Ne}\right)$$

worin

Ne, Nc bzw. No die Refraktionsindizes der Eintrittslinse (4), der Kontaktlinse (5) bzw. des Auges (3) bezeichnen ;

Re, Rc bzw. Ro die Krümmungsradien der Eintrittsfläche (8), eines von der Eintrittslinse (4) und der Kontaktlinse (5) gebildeten Diopters bzw. der Kontaktfläche bezeichnen ;

d den Abstand zwischen dem Arbeitspunkt (T) und der Kontaktfläche in der optischen Achse (6) des Glases (1) bezeichnet und

ee bzw. ec die Abstände in der optischen Achse (6) des Glases (1) zwischen der Eintrittsfläche (8) und dem Diopter bzw. dem Diopter und der Kontaktfläche bezeichnen.

4. Kontaktglas nach Anspruch 3, dadurch gekennzeichnet, daß der Krümmungsradius Re durch die genannte Beziehung definiert ist, in welcher

$7\ mm \leq Ro \leq 9\ mm$ Tabulae Biologicae
$1{,}33 \leq No \leq 1{,}34$ Tabulae Biologicae
$0{,}5\ mm \leq ec \leq 30\ mm$ Tabulae Biologicae
$1{,}3 \leq Nc \leq 2{,}0$ Tabulae Biologicae
$2\ mm \leq Re \leq 100\ mm$ Tabulae Biologicae
$1{,}3 \leq Ne \leq 2{,}0$ Tabulae Biologicae
$0{,}5\ mm \leq ee \leq 30\ mm$ Tabulae Biologicae
$0{,}5\ mm \leq d \leq 30\ mm$ Tabulae Biologicae

5. Kontaktglas nach einem der vorangehenden Ansprüche 2 bis 4, dadurch gekennzeichnet, daß die Eintrittslinse (4) aus mineralischem Glas besteht und die Kontaktlinse (5) aus einem organischen Material.

6. Kontaktglas nach Anspruch 5, dadurch gekennzeichnet, daß die Eintrittsfläche (8) mit einer Reflex mindernden Beschichtung versehen ist.

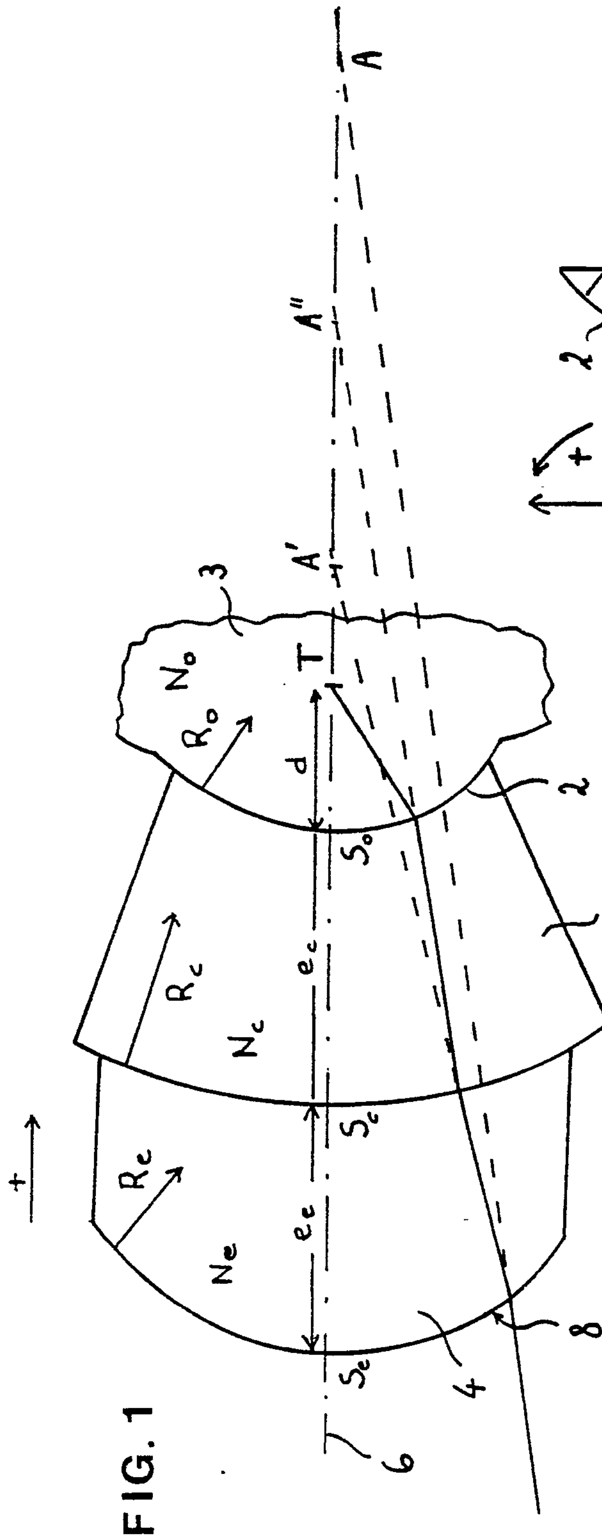


FIG. 1

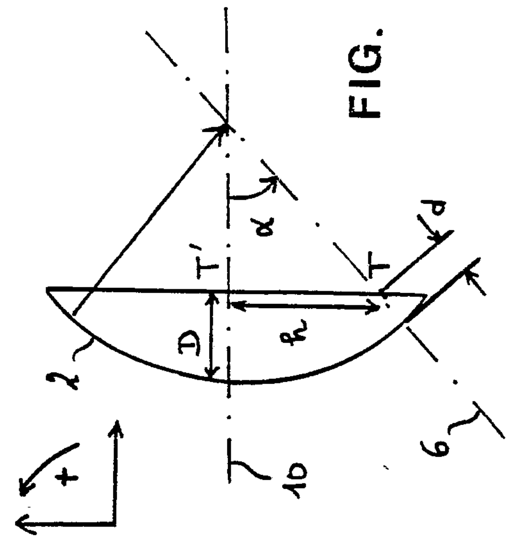


FIG. 2

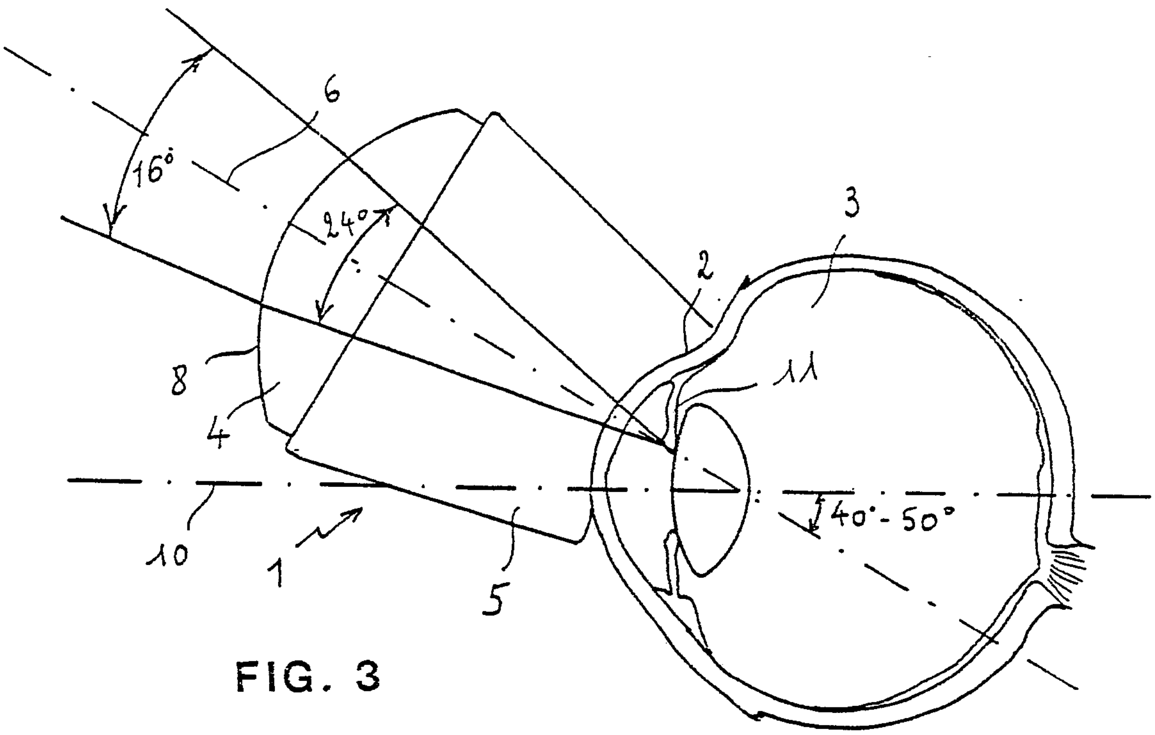


FIG. 3

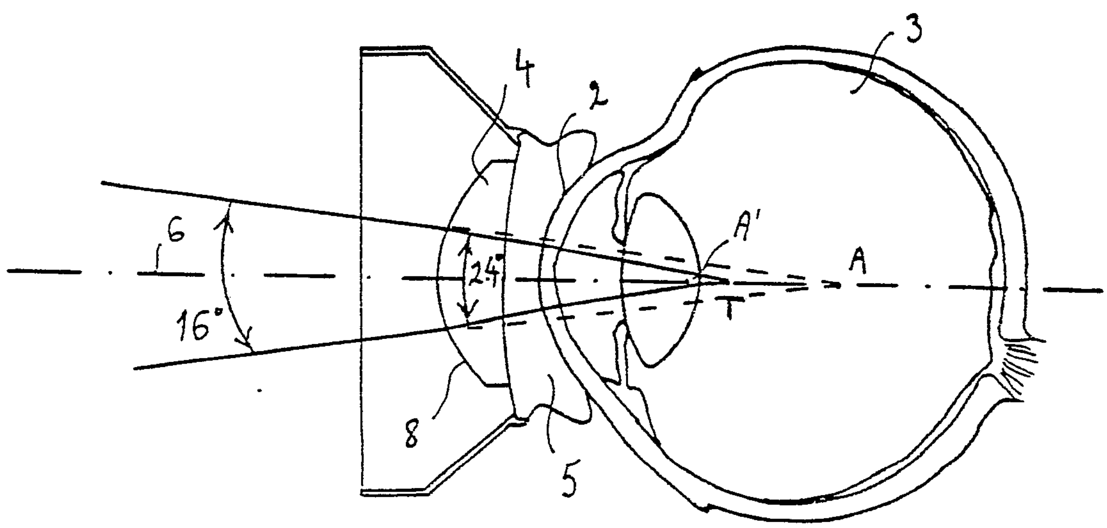


FIG. 4